(19) 

Europäisches Patentamt

European Patent Office

Office européen des brevets

(11) **EP 4 425 001 A1**

(12) **EUROPEAN PATENT APPLICATION**

(43) Date of publication:
**04.09.2024 Bulletin 2024/36**

(21) Application number: **24156151.3**

(22) Date of filing: **06.02.2024**

(51) International Patent Classification (IPC):
**F15D 1/02** *(2006.01)* **G01F 15/18** *(2006.01)*
**G01N 33/00** *(2006.01)* **G08B 29/20** *(2006.01)*

(52) Cooperative Patent Classification (CPC):
**G01F 15/185; F15D 1/02; F15D 1/025;**
**G01N 33/0006; G01N 33/0009;** G01N 33/0011;
G01N 33/0016; G08B 29/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **02.03.2023 CN 202310193910**

(71) Applicant: **Honeywell International Inc.**
**Charlotte, NC 28202 (US)**

(72) Inventors:
- YANG, Fan
  **Charlotte, 28202 (US)**
- WANG, Houyong
  **Charlotte, 28202 (US)**
- HU, Yu
  **Charlotte, 28202 (US)**
- LIANG, Feng
  **Charlotte, 28202 (US)**

(74) Representative: **Haseltine Lake Kempner LLP**
**Cheapside House**
**138 Cheapside**
**London EC2V 6BJ (GB)**

(54) **FIXTURE FOR SMOOTHING GAS FLOW TO GAS SENSOR**

(57) A fixture for smoothing a gas flow to a gas sensor is provided. The fixture includes a cap structure defining a flow channel, where the flow channel includes a chamber and an inlet connected to the chamber, and the flow channel is configured to transport the gas flow to the gas sensor; a first grid layer including a plurality of first openings and located in the chamber; and a second grid layer including a plurality of second openings and located in the chamber, where the gas flow is smoothed by passing through the first grid layer through the plurality of first openings and the second grid layer through the plurality of first openings sequentially.

FIG. 1

EP 4 425 001 A1

## Description

## FIELD OF THE INVENTION

Exemplary embodiments of the present disclosure relate generally to a fixture for smoothing gas flow, and more particularly, in some examples, to a fixture for smoothing gas flow during a gas sensor calibration process.

## BACKGROUND

Multiple gas flow sensors may be calibrated simultaneously to improve efficiency. Vent caps are designed to send a same volume of gas to each gas flow sensor. However, gas flow sensors located at different locations, in some examples, may receive different volumes, velocities and directions of gas flow due to manufacture errors of the vent caps and housings of the gas flow sensors. Through applied effort, ingenuity, and innovation, many of these identified problems have been solved by developing solutions that are included in embodiments of the present disclosure, many examples of which are described in detail herein.

## BRIEF SUMMARY

Various embodiments described herein relate to methods, apparatuses, and systems for improving sensitivity and performance of an apparatus such as, for example, a flow sensing apparatus.

In accordance with various examples of the present disclosure, a fixture for smoothing a gas flow to a gas sensor is provided. The fixture includes a cap structure defining a flow channel, where the flow channel includes a chamber and an inlet connected to the chamber, and the flow channel is configured to transport the gas flow to the gas sensor; a first grid layer including a plurality of first openings and located in the chamber; and a second grid layer including a plurality of second openings and located in the chamber, where the gas flow is smoothed by passing through the first grid layer through the plurality of first openings and the second grid layer through the plurality of first openings sequentially.

In some embodiments, a distance between the first grid layer and the second grid layer is greater than a thickness of the first grid layer.

In some embodiments, the plurality of first openings are distributed in an array in the first grid layer.

In some embodiments, a shape of a projection of each of plurality of first openings on the first grid layer along a flow direction of the gas flow is a circle, an ellipse, or a kidney slot.

In some embodiments, the plurality of second openings are distributed in an array in the second grid layer.

In some embodiments, a shape of a projection of each of plurality of second openings on the first grid layer along a flow direction of the gas flow is a circle, an ellipse, or a kidney slot.

In some embodiments, an overlap area between a projection of the plurality of first openings on the second grid layer along a flow direction of the gas flow and the plurality of second openings is less than 10% of an area of the projection of the plurality of first openings on the second grid layer along the flow direction of the gas flow.

In some embodiments, the projection of the plurality of first openings on the second grid layer along the flow direction of the gas flow has no overlap with the plurality of second openings.

In some embodiments, the cap structure is made of a plastic.

In some embodiments, the plastic is poly(methyl methacrylate) (PMMA), cyclic olefine copolymers, polycarbonate, polystyrene, polyether ether ketone (PEEK), polytetrafluoroethylene (PTFE), liquid-crystal polymers (LCPs), polyetherimide (PEI), epoxy, PerFluoroAlkoxy (PFA), fluorinated ethylene propylene (FEP), or a combinations thereof.

In some embodiments, the gas sensor is an electro chemical sensor (ECS) configured to measure a concentrations of a target particle in the gas flow.

In some embodiments, the chamber takes a form of a cylindrical tube.

In some embodiments, the inlet takes a form of a cylindrical tube.

In some example embodiments, a fixture for smoothing a gas flow to a gas sensor is provided. The fixture includes: a cap structure defining a flow channel, where the flow channel includes a cylindrical chamber and an inlet connected to the cylindrical chamber, and the flow channel is configured to transport the gas flow to the gas sensor, a height of the cylindrical chamber is a function of a height of the gas sensor, and a diameter of the cylindrical chamber is a function of a diameter of the gas sensor.

In some embodiments, the height of the cylindrical chamber is defined according to $0.5*Hs < H_f < 1.5*Hs$ where the height of the cylindrical chamber is $H_f$ and the height of the gas sensor is $H_s$.

In some embodiments, the diameter of the cylindrical chamber is defined according to $0.5*Ds < D_f < 1.5*Ds$ where the diameter of the cylindrical chamber is $D_f$, and the diameter of the gas sensor is $D_s$.

In some embodiments, the cap structure is made of a plastic.

In some embodiments, the plastic is poly(methyl methacrylate) (PMMA), cyclic olefine copolymers, polycarbonate, polystyrene, polyether ether ketone (PEEK), polytetrafluoroethylene (PTFE), liquid-crystal polymers (LCPs), polyetherimide (PEI), epoxy, PerFluoroAlkoxy (PFA), fluorinated ethylene propylene (FEP), or a combinations thereof.

In some embodiments, the gas sensor is an electro chemical sensor (ECS) configured to measure a concentrations of a target particle in the gas flow.

In some embodiments, the inlet takes a form of a cylindrical tube.

## BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:

> FIG. 1 illustrates a schematic diagram depicting a sectional view of an example fixture for smoothing a gas flow to a gas sensor in accordance with various embodiments of the present disclosure;
> FIG. 2 illustrates a schematic diagram depicting a top view of a first grid layer and a top view of a second grid layer in accordance with various embodiments of the present disclosure;
> FIG. 3 illustrates a schematic diagram depicting a sectional view of an example fixture for smoothing a gas flow to a gas sensor in accordance with various embodiments of the present disclosure; and
> FIG. 4 illustrates a schematic diagram depicting a sectional view of an apparatus for smoothing a gas flow to a plurality of gas sensors in accordance with various embodiments of the present disclosure.

## DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in a dashed line for visibility of the underlying components.

The phrases "in an example embodiment," "some embodiments," "various embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such components or features may be optionally included in some embodiments, or may be excluded.

The term "electronically coupled" or "in electronic communication with" in the present disclosure refers to two or more electrical elements (for example, but not limited to, an example processing circuitry, communication module, input/output module memory, humidity sensing component, cooling element, gas detection component) and/or electric circuit(s) being connected through wired means (for example but not limited to, conductive wires or traces) and/or wireless means (for example but not limited to, wireless network, electromagnetic field), such that data and/or information (for example, electronic indications, signals) may be transmitted to and/or received from the electrical elements and/or electric circuit(s) that are electronically coupled.

The term "gas sensor" may refer to a sensor that may detect, measure, and/or identify concentrations of particles of a flowing media or medium. For example, the gas sensor may be an electro chemical sensor (ECS) designed to measure concentrations of various gases. In some examples, the gas sensor may measure concentration of target gas particles, such as particles related to explosive gases (e.g., propane) and/or toxic gases (e.g., carbon monoxide). In some examples, and as described herein, the gas sensor may utilize or otherwise rely on a linear relationship between the concentration of the target gas particle and the output current of the gas sensor to measure the concentration of the target gas particle. As such, the gas sensor may, in some examples, calculate a concentration of a target gas particle.

In the present disclosure, the term "flowing media" refers to a substance, such as, but not limited to, a liquid substance and/or a gaseous substance, such as air. In some examples, flowing media may include one or more gases that are flowing through a structure, enclosure and/or the like.

In some examples described herein and in order to improve the accuracy of the measurement, a gas sensor may need to be calibrated before use and/or may need to be calibrated during use. During calibration and by way of example, a gas flow, with a known concentration of a target gas particle, may be provided or otherwise injected into a gas sensor through a vent cap and into a flow channel. Thus, by calibrating the gas sensor output current with a known concentration of the target gas particle, the gas sensor can be used for quantitative measurements of the concentration of the target gas particle. In some examples, the gas sensor may be in electronic communication with electrical elements (for example, but not limited to, an example processing circuitry, communication module, input/output module memory), and gas sensor may be calibrated according the measurement of the concentration of the target gas particle and the known concentration of the target gas particle.

However, in some examples, the measurement of the concentration of the target gas particle may be affected by how even the gas flows through the flow channel (e.g., evenness of a gas flow). In some examples, the term "velocity evenness" of a gas flow may refer to a velocity distribution of the gas flow in a flow channel (e.g., a transportation channel). For example, the velocity evenness of a gas flow is greater when the flow rates of the gas particles are evenly distributed in the flow channel. The velocity evenness of the gas flow may be affected by the shape and the size of the transportation channel, and the flow direction of the gas flow, the vent cap, and/or the like.

In some examples, the calibration process may rely on multiple sensors being calibrated at the same time and may, in some examples, rely on vent caps to make sure all sensors receive the same or substantially the same volume of gas. However, errors may lead to different flow rates. For example, errors or other imperfections during manufacturing of a vent cap or a sensor housing may influence or otherwise affect the relative location / distance between a gas inlet and sensor, which may, lead to difference in gas flow rate. Indeed, parts manufacture error and assembly error may impact gas flow rate and direction, which may, in some examples, lead to different sensitivity even testing the same sensor. In some examples and in an instance in which sensor yield rates are impacted, sensor manufacture cost may be higher.

In some examples and as described herein, a fixture including a vent cap structure may be utilized in a variety of applications to smooth or otherwise even the gas flow and improve the evenness of the gas flow. For example, a vent cap structure, such as the vent cap structure disclosed herein, may define a flow channel to transport the gas flow to the gas sensor, such that the velocity evenness of the gas flow is improved. In some examples, the fixture may further include a first grid layer with a plurality of first openings and a second grid layer with a plurality of second openings located in the flow channel to smooth or otherwise even the gas flow. In some examples, an overlap area between a projection of the plurality of first openings on the second grid layer along a direction of the gas flow into a flow channel and the plurality of second openings is less than 25% of an area of the plurality of first openings, less than 15% of an area of the plurality of first openings, and/or less than 10% of an area of the plurality of first openings.

In some examples, the flow channel may include a cylindrical chamber and an inlet connected to the cylindrical chamber and is configured to transport the gas flow to the gas sensor. In some examples, the cylindrical chamber may be sized as a function of the height and the diameter of a gas sensor. For example, a height of the cylindrical chamber is $H_f$, a diameter of the cylindrical chamber is $D_f$, a height of the gas sensor is $H_s$, a diameter of the gas sensor is $D_s$, where $0.5*H_s < H_f < 1.5*H_s$, and $0.5*D_s < D_f < 1.5*D_s$.

In view of the disclosures herein and in some examples, the present disclosure may improve performance, sensitivity, and/or accuracy, of calibrating a gas sensor, and/or may, in some examples, enable measurement of a concentration of a target particle in a gas flow. In another example, the techniques and the fixtures described herein may be used to smooth or otherwise even the gas flow to each gas sensor without introducing too much flow resistance and multiple sensors may be calibrated at the same time, such that efficiency of the calibration process can be improved.

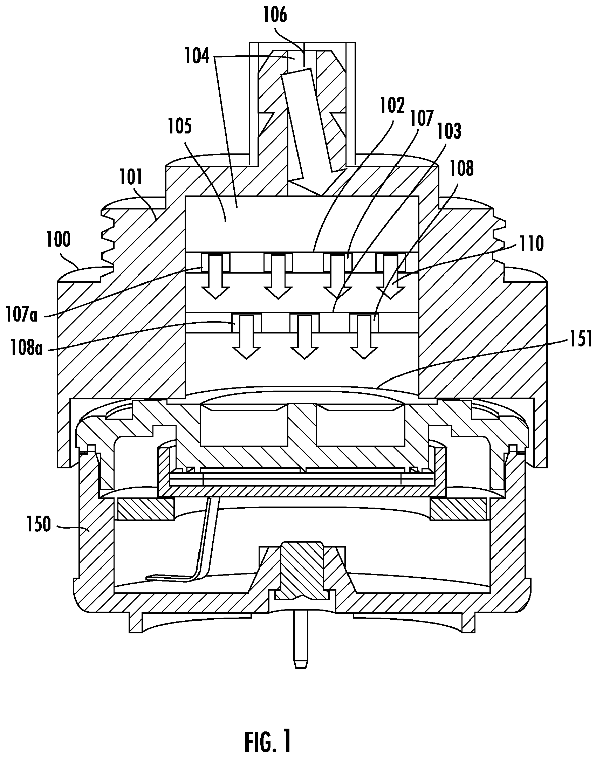

Referring now to FIG. 1, a schematic diagram depicting a sectional view of an example fixture for smoothing or otherwise evening a gas flow to a gas sensor in accordance with various embodiments of the present disclosure is provided. As depicted in FIG. 1, a fixture 100 includes a cap structure 101, a first grid layer 102 in the cap structure 101, and a second grid layer 103 in the cap structure 101.

As illustrated in FIG. 1, the cap structure 101 defines a flow channel 104 configured to transport a gas flow from an external source to a gas sensor 150, such as during calibration. For example, the flow channel 104 may include a chamber 105 and an inlet 106 connecting with the chamber 105 to transport the gas flow from the external source to the gas sensor 150.

In various embodiments, the chamber 105 is formed by internal walls of the cap structure 101. In some examples, the chamber 105 may be configured to be disposed adjacent the gas sensor 150 during a calibration process, such that the gas flows directly to the gas sensor 150 from the chamber 105 and a concentration of a target particle in the gas flow may be measured by the gas sensor. In some examples, the chamber 105 may be disposed adjacent to the gas sensor 150 during a calibration process, such that an interface surface 151 of gas sensor may be exposed to the gas flow in order to measure the concentration of the target particle in the gas flow. For example, the interface surface 151 of the gas sensor 150 may be a thin filter film to allow the target particle to go through and be measured by the gas sensor 150.

In various embodiments, the gas sensor 150 may detect, measure, and/or identify concentrations of particles of a flowing media or medium. For example, the gas sensor 150 may be an electro chemical sensor (ECS) designed to measure concentrations of various gases. In some examples, the gas sensor may measure concentration of target gas particles, such as particles related to explosive gases (e.g., propane) and/or toxic gases (e.g., carbon monoxide). In some examples, and as described herein, the gas sensor may utilize or otherwise rely on a linear relationship between the concentration of the target gas particle and the output current of the gas sensor to measure the concentration of the target gas particle. As such, the gas sensor may, in some examples, calculate a concentration of a target gas particle.

In various embodiments, the inlet 106 may take a form of a tubular shape. For example, the inlet may be a cylindrical tube to transport the gas flow from the external source to the chamber 105.

In various embodiments, the chamber 105 may take a form of a tubular shape. For example, the chamber 105 may be a cylindrical tube, configured to transport the gas flow from the inlet 106 to the gas sensor 150.

In various embodiments, the first grid layer 102 and the second grid layer 103 may be located in the chamber 105. In some examples, the first grid layer 102 and the second grid layer 103 may be spaced apart from each other. In some examples, the first grid layer 102 and the second grid layer 103 may have a same thickness. For example, a distance between the first grid layer 102 and the second grid layer 103 may be greater than a thickness of the first grid layer 102. For example, the distance between the first grid layer 102 and the second grid layer 103 may be greater than a thickness of the second grid layer 103.

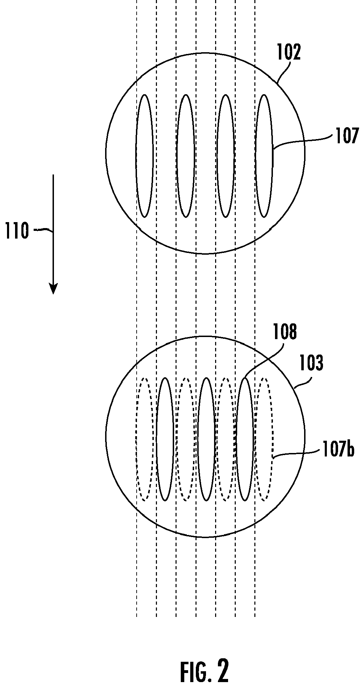

In various embodiments, the first grid layer 102 may include a plurality of first openings 107, such that the gas flow may pass through the first grid layer 102 through the plurality of first openings 107. In some embodiments, the plurality of first openings 107 are uniformly distributed in an array in the first grid layer 102. For example, the plurality of first openings 107 may be distributed in a one-dimensional array or a two-dimensional array. For example, as shown in FIG. 2, the plurality of first openings 107 are evenly distributed in a one-dimensional array. In some embodiments, a sidewall 107a of each first openings 107 is extending along a flow direction 110 of the gas flow.

In various embodiments, the second grid layer 103 may include a plurality of second openings 108, such that the gas flow may pass through the second grid layer 103 through the plurality of second openings 108 after the gas flow passes through the first grid layer 102. In some embodiments, the plurality of second openings 108 are uniformly distributed in an array in the second grid layer 103. For example, the plurality of second openings 108 may be distributed in a one-dimensional array or a two-dimensional array. For example, as shown in FIG. 2, the plurality of first openings 108 are evenly distributed in a one-dimensional array. In some embodiments, a sidewall 108a of each second openings 108 is extending along the flow direction 110 of the gas flow.

In various embodiments, the first grid layer 102 and the second grid layer 103 are stacked in the chamber 105, such that the gas flow may be transported through the first grid layer 102 and the second grid layer 103 sequentially during a calibration process. In some examples, the plurality of first openings 107 and the plurality of second openings 108 are configured to guide the gas flow along the flow direction 110, such that the velocity evenness of the gas flow may be improved after passing through the first grid layer 102 and the second grid layer 103.

In various embodiments, the fixture 100 may be made of a plastic. For example, the fixture 100 may be made of, poly(methyl methacrylate) (PMMA), cyclic olefine copolymers, polycarbonate, polystyrene, polyether ether ketone (PEEK), polytetrafluoroethylene (PTFE), liquid-crystal polymers (LCPs), polyetherimide (PEI), epoxy, PerFluoroAlkoxy (PFA), fluorinated ethylene propylene (FEP), combinations thereof, and/or the like.

While the description above provides an example fixture 100, it is noted that the scope of the present disclosure is not limited to the description above. In some examples, an example fixture 100 in accordance with the present disclosure may be in other forms. In some examples, the fixture 100 may include one or more additional and/or alternative elements, and/or may be structured/positioned differently than that illustrated in FIG. 1.

Referring now to FIG. 2, a schematic diagram depicting a top view of the first grid layer and a top view of the second grid layer in accordance with various embodiments of the present disclosure is provided. For illustration purpose, the top view of the first grid layer and the top view of the second grid layer are aligned along a flow direction of the gas flow.

As depicted in FIG. 2, a top view of the first grid layer 102 and a top view of the second grid layer 103 from FIG. 1 are aligned along the flow direction 110 of the gas flow. In some embodiments, the first grid layer 102 may include a plurality of first openings 107, and the second grid layer 103 may include a plurality of second openings 108. For example, a shape of each of plurality of first openings 107 on the first grid layer 102 is a circle, an ellipse, a kidney slot, and/or the like. In some embodiments, the shape of the first openings 107 may be any other shapes without sharp corners. For example, a shape of each of plurality of second openings 108 on the second grid layer 103 is a circle, an ellipse, a kidney slot, and/or the like. In some embodiments, the shape of the second openings 108 may be any other shapes without sharp corners.

In various embodiments, the plurality of first openings 107 are uniformly distributed in an array on the first grid layer 102. In various embodiments, the plurality of second openings 108 are uniformly distributed in an array on the second grid layer 103. In some examples, an overlap between the plurality of first openings 107 and the plurality of second openings 108 is less than 25% of an area of the plurality of first openings, less than 15% of an area of the plurality of first openings, and/or less than 10% of an area of the plurality of first openings.

In various embodiments, an overlap area between the projection 107b of the plurality of first openings 107 on the second grid layer 103 along the flow direction 110 and the plurality of second openings 108 is less than 10% of an area of the plurality of first openings 107. In some examples, there is no overlap between the projection of the plurality of first openings 107 on the second grid layer 103 along the flow direction 110 and the plurality of second openings 108.

In some examples and with the above overlap configuration, the gas flow is guided along the flow direction 110 and regulated by the first grid layer 102 and the second grid layer 103 sequentially. For that reason, the velocity evenness of the gas flow may be improved and the accuracy of calibration of gas sensor with the regulated gas flow may be improved. In addition, there are very limited height requirements for the chamber 105 by using the first grid layer 102 and the second grid layer 103 to guide and regulate the gas flow.

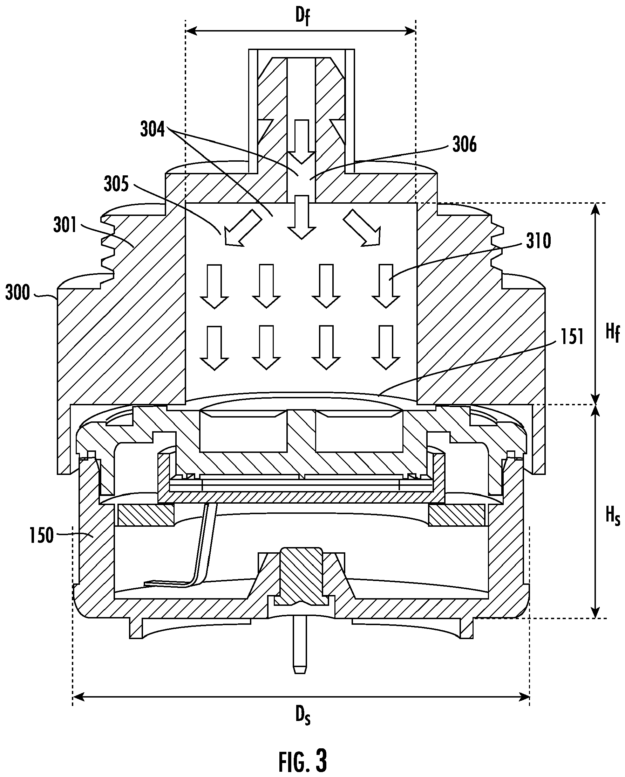

Referring now to FIG. 3, a schematic diagram depicting a sectional view of an example fixture for smoothing or otherwise evening a gas flow to a gas sensor in accordance with various embodiments of the present disclosure is provided.

As illustrated in FIG. 3, a fixture 300 includes a cap structure 301 and the cap structure 301 defines a flow channel 304 configured to transport a gas flow to a gas sensor 150 during a calibration process. For example, the flow channel 304 may include a chamber 305 and an inlet 306 connecting with the chamber 305 to transport the gas flow to the gas sensor 150.

In various embodiments, the chamber 305 is formed by internal walls of the cap structure 301. In some examples, the chamber 305 may be configured to be disposed adjacent the gas sensor 150 during a calibration process, such that the gas flow may be transported to the gas sensor 150 through the chamber 305 and a concentration of a target particle in the gas flow may be measured by the gas sensor 150. In some examples, the chamber 305 may be disposed adjacent to the gas sensor 150 during a calibration process, such that an interface surface 151 of gas sensor 150 may be exposed to the gas flow in order to measure the concentration of the target particle in the gas flow. For example, the interface surface 151 of the gas sensor 150 may be a thin filter film to allow the target particle to go through and be measured by the gas sensor 150.

In various embodiments, the inlet 306 may take a form of a tubular shape. For example, the inlet 306 may be a cylindrical tube to transport the gas flow from an external source to the chamber 305.

In various embodiments, the chamber 305 may take a form of a tubular shape. For example, the chamber 305 may be a cylindrical tube to transport the gas flow from the inlet 306 to the gas sensor 150. As illustrated in FIG. 3, a height of the cylindrical chamber is a function of the height and/or diameter of the sensor itself. For example, a height of the cylindrical chamber 305 is $H_f$, a diameter of the cylindrical chamber 305 is $D_f$, a height of the gas sensor 150 is $H_s$, and a diameter of the gas sensor 150 is $D_s$. In some embodiments, the height of the cylindrical chamber $H_f$ is a function of the height of the gas sensor $H_s$ and the diameter of the cylindrical chamber $D_f$ is a function of the diameter of the gas sensor $D_s$. In some examples, the size of the chamber is adjusted according to the size of the gas sensor, such that the gas flow is guided along the flow direction 310 and the chamber 305 of the fixture 300 has enough space to regulate the gas flow. For that reason, the velocity evenness of the gas flow may be improved and the accuracy of calibration of gas sensor with the regulated gas flow may be improved (e.g., sensor response time).

For example, the height of the cylindrical chamber $H_f$ and the diameter of the cylindrical chamber $D_f$ may satisfy following inequalities:

$$0.5*Hs < H_f < 1.5*Hs,$$

and

$$0.5*Ds < D_f < 1.5*Ds.$$

It is noted that if the height of the cylindrical chamber $H_f$ is too big with respect to the height of the gas sensor is $H_s$ or the diameter of the cylindrical chamber $D_f$ is too big with respect to the diameter of the gas sensor $D_s$, a response time of the gas sensor 150 to the target particle may, in some examples, be affected. Similarly, if the height of the cylindrical chamber $H_f$ is too small with respect to the height of the gas sensor is $H_s$ or the diameter of the cylindrical chamber $D_f$ is too small with respect to the diameter of the gas sensor $D_s$, regulation effects of the cylindrical chamber 305 on the gas flow may, in some examples, be decreased and the velocity evenness of the gas flow may, in some examples, not be improved.

In various embodiments, the fixture 300 may be made of a plastic. For example, the fixture 300 may be made of, poly(methyl methacrylate) (PMMA), cyclic olefine copolymers, polycarbonate, polystyrene, polyether ether ketone (PEEK), polytetrafluoroethylene (PTFE), liquid-crystal polymers (LCPs), polyetherimide (PEI), epoxy, PerFluoroAlkoxy (PFA), fluorinated ethylene propylene (FEP), combinations thereof, and/or the like.

While the description above provides an example fixture 300, it is noted that the scope of the present disclosure is not limited to the description above. In some examples, an example fixture 300 in accordance with the present disclosure may be in other forms. In some examples, an fixture 300 may include one or more additional and/or alternative elements, and/or may be structured/positioned differently than that illustrated in FIG. 3.

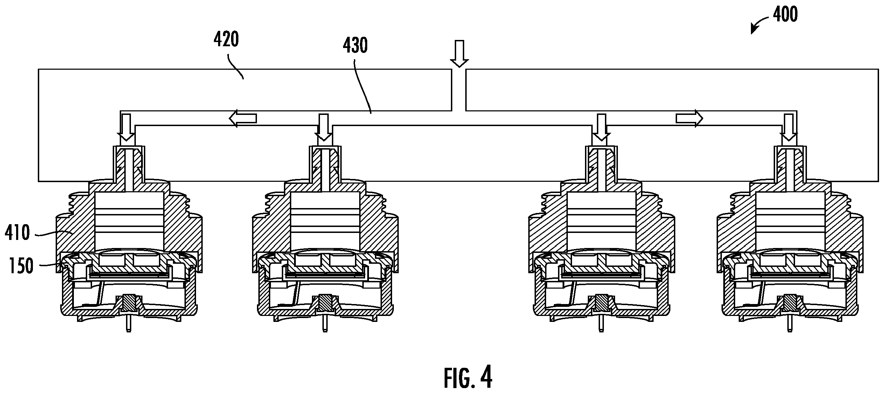

Referring now to FIG. 4, a schematic diagram depicting a sectional view of an apparatus 400 using of example fixtures for smoothing or otherwise evening a gas flow to a plurality of gas sensors in accordance with various embodiments of the present disclosure is provided.

As depicted in FIG. 4, the apparatus 400 may be used to calibrate a plurality of gas sensors simultaneously. The apparatus 400 may include a vent cap 420 to define pipes 430, such that a gas flow may be transported from an external source to the plurality of gas sensors 150 through a corresponding fixture 410 during a calibration process.

In some embodiments, the vent cap 420 and the plurality of fixtures 410 may be integrated into a single part. In some embodiments, the vent cap 420 and the plurality of fixtures 410 may be separated parts.

For illustration purpose, each of the plurality of fixtures 410 in apparatus 400 is a fixture 100 as described in FIG. 1. In some embodiments, the fixture 410 in apparatus 400 may be a fixture 300 as described in FIG. 3.

In some examples and with the above apparatus 400, a plurality of gas sensors may be calibrated simultaneously while the gas flow to each of the plurality of gas sensors are smoothed and evenly distributed. For that reason, the accuracy and the efficiency of the calibration of gas sensors may be improved.

While the description above provides an example apparatus 400, it is noted that the scope of the present disclosure is not limited to the description above. In some examples, an example apparatus 400 in accordance with the present disclosure may be in other forms. In some examples, an apparatus 400 may include one or more additional and/or alternative elements, and/or may be structured/positioned differently than that illustrated in FIG. 4.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A fixture for smoothing a gas flow to a gas sensor, comprising:

   a cap structure defining a flow channel, wherein the flow channel includes a chamber and an inlet connected to the chamber, and the flow channel is configured to transport the gas flow to the gas sensor;
   a first grid layer including a plurality of first openings and located in the chamber; and
   a second grid layer including a plurality of second openings and located in the chamber, wherein the gas flow is smoothed by passing through the first grid layer through the plurality of first openings and the second grid layer through the plurality of first openings sequentially.

2. The fixture according to claim 1, wherein a distance between the first grid layer and the second grid layer is greater than a thickness of the first grid layer.

3. The fixture according to claim 1, wherein the plurality of first openings are distributed in an array in the first grid layer.

4. The fixture according to claim 1, wherein a shape of a projection of each of plurality of first openings on the first grid layer along a flow direction of the gas flow is a circle, an ellipse, or a kidney slot.

5. The fixture according to claim 1, wherein the plurality of second openings are distributed in an array in the second grid layer.

6. The fixture according to claim 1, wherein a shape of a projection of each of plurality of second openings on the first grid layer along a flow direction of the gas flow is a circle, an ellipse, or a kidney slot.

7. The fixture according to claim 1, wherein an overlap area between a projection of the plurality of first openings on the second grid layer along a flow direction of the gas flow and the plurality of second openings is less than 10% of an area of the projection of the plurality of first openings on the second grid layer along the flow direction of the gas flow.

8. The fixture according to claim 7, wherein the projection of the plurality of first openings on the second grid layer along the flow direction of the gas flow has no overlap with the plurality of second openings.

9. The fixture according to claim 1, wherein the cap structure is made of a plastic.

10. The fixture according to claim 9, wherein the plastic is poly(methyl methacrylate) (PMMA), cyclic olefine copolymers, polycarbonate, polystyrene, polyether ether ketone (PEEK), polytetrafluoroethylene (PTFE), liquid-crystal polymers (LCPs), polyetherimide (PEI), epoxy, PerFluoroAlkoxy (PFA), fluorinated ethylene propylene (FEP), or a combinations thereof.

11. The fixture according to claim 1, wherein the gas sensor is an electro chemical sensor (ECS) configured to measure a concentrations of a target particle in the gas flow.

12. The fixture according to claim 1, wherein the chamber takes a form of a cylindrical tube.

13. The fixture according to claim 1, wherein the inlet takes a form of a cylindrical tube.

14. A fixture for smoothing a gas flow to a gas sensor, comprising:
a cap structure defining a flow channel, wherein:

   the flow channel includes a cylindrical chamber and an inlet connected to the cylindrical chamber, and the flow channel is configured to transport the gas flow to the gas sensor,
   a height of the cylindrical chamber is a function of a height of the gas sensor, and
   a diameter of the cylindrical chamber is a function of a diameter of the gas sensor.

15. The fixture according to claim 14, wherein the height of the cylindrical chamber is defined according to $0.5*Hs < H_f < 1.5*Hs$ where the height of the cylindrical chamber is $H_f$ and the height of the gas sensor is $H_s$.

FIG. 1

FIG. 2

FIG. 3

FIG. 4

Europäisches
Patentamt

European
Patent Office

Office européen
des brevets

# EUROPEAN SEARCH REPORT

Application Number

EP 24 15 6151

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 39 40 034 A1 (ROSEMOUNT GMBH & CO [DE]) 6 June 1991 (1991-06-06) * column 2, lines 3-65 * * column 3, line 23 - column 4, line 38; figures 1-5 * | 1-15 | INV. F15D1/02 G01F15/18 G01N33/00 ADD. G08B29/20 |
| X | CA 3 189 389 A1 (NISSHINBO HOLDINGS INC [JP]; JAPAN RADIO UEDA CO LTD [JP] ET AL.) 27 January 2022 (2022-01-27) * page 2, line 11 - page 3, line 27 * * page 4, line 28 - page 14, line 10; figures 1-7 * | 1-13 | |
| X | US 2006/162426 A1 (LANGE BJORN [DE] ET AL) 27 July 2006 (2006-07-27) * paragraphs [0003], [0005] - [0009], [0021] - [0025]; figures 1-3 * | 14,15 9-11 | |
| X A | THORSTEN GRAUNKE ET AL: "Organic Membranes for Selectivity Enhancement of Metal Oxide Gas Sensors", JOURNAL OF SENSORS, vol. 2016, 28 July 2016 (2016-07-28), pages 1-22, XP055369909, US ISSN: 1687-725X, DOI: 10.1155/2016/2435945 * pages 1,11,12; figure 4 * | 14 9-11 | TECHNICAL FIELDS SEARCHED (IPC) F15D G01F G08B G01N |

The present search report has been drawn up for all claims

| Place of search | Date of completion of the search | Examiner |
|---|---|---|
| The Hague | 8 July 2024 | Myrillas, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 6151

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2024

| Patent document cited in search report | | Publication date | Patent family member(s) | | Publication date |
|---|---|---|---|---|---|
| DE 3940034 | A1 | 06-06-1991 | DE | 3940034 A1 | 06-06-1991 |
| | | | EP | 0433741 A1 | 26-06-1991 |
| CA 3189389 | A1 | 27-01-2022 | CA | 3189389 A1 | 27-01-2022 |
| | | | CN | 116134310 A | 16-05-2023 |
| | | | EP | 4187241 A1 | 31-05-2023 |
| | | | JP | 7451336 B2 | 18-03-2024 |
| | | | JP | 2022021408 A | 03-02-2022 |
| | | | KR | 20230039660 A | 21-03-2023 |
| | | | US | 2023258207 A1 | 17-08-2023 |
| | | | WO | 2022019276 A1 | 27-01-2022 |
| US 2006162426 | A1 | 27-07-2006 | DE | 102005003050 B3 | 29-06-2006 |
| | | | GB | 2422432 A | 26-07-2006 |
| | | | US | 2006162426 A1 | 27-07-2006 |

EPO FORM P0459

For more details about this annex : see Official Journal of the European Patent Office, No. 12/82